# DEMANDE DE BREVET EUROPEEN

(11) **EP 2 597 461 A2**
(43) Date de publication de la demande: **29.05.2013**
(21) Numéro de dépôt: 12193621.5
(22) Date de dépôt: 21.11.2012
(51) Int. Cl.: G01N 33/18, C02F 3/30, C12M 1/34

(54) **Procédé de mesure directe de multiples biodégradabilités**

(30) Priorité: 23.11.2011 FR 1160685
(71) Demandeur: Envolure, 34790 Grabels (FR)
(72) Inventeur: Pautremat, Nathalie, 34070 Montpellier (FR); Goy, Romy-Alice, 34400 Saint-Just (FR); El Amraoui, Zaynab, 34080 Montpellier (FR); Dudal, Yves, 68220 Hegenheim (FR)
(74) Mandataire: Pontet Allano & Associes

(57) **Abrégé**

La présente invention concerne un procédé de mesure de la biodégradabilité des substrats organiques par la détection fluorescente et/ou colorimétrique de l'activité microbienne générée par l'ajout de substrats organiques dans un mélange de micro-organismes.

## Description

La présente invention concerne un procédé de mesure de biodégradabilité de substrats organiques basé sur l'utilisation d'un bioréactif fluorescent et/ou colorimétrique pour évaluer l'activité microbienne générée par l'ajout de substrats organiques dans un mélange de micro-organismes. Ce procédé a été développé pour permettre des mesures aussi bien en conditions aérobies qu'anaérobies.

L'invention s'applique au domaine de l'analyse de la biodégradabilité de matières organiques de types variés, tels que, par exemple, eaux et boues de station d'épuration, effluents agro-industriels ou d'élevage, déchets industriels, agro-industriels et agricoles ou compost.

Les substrats organiques sont utilisables par les micro-organismes en tant que substrats desquels ils retirent de l'énergie et la capacité de croître. De nombreuses applications industrielles utilisent ces réactions biochimiques de dégradation, en conditions aérobies ou anaérobies, notamment : épuration des eaux usées, production de composés à haute valeur ajoutée, production d'énergie, et production agro-alimentaire. Les sources de substrats organiques disponibles sont très variées : cultures et résidus de culture, production et résidus agro-alimentaires, déchets ménagers et urbains, biomasse notamment algale.

Il existe différentes méthodes pour mesurer la biodégradabilité des substrats organiques, c'est-à-dire pour mesurer l'étendue avec laquelle des micro-organismes vont pouvoir utiliser ces substrats pour les transformer. Chaque domaine d'application utilise sa propre méthode très généralement basée sur la mesure du produit formé au cours de la réaction, parfois sur la mesure du substrat dégradé. Ces méthodes sont longues, complexes à mettre en place et requièrent un appareillage lourd et coûteux.

Deux paramètres industriels sont plus couramment utilisés pour mesurer la biodégradabilité des substrats organiques : la demande biochimique (ou biologique) en oxygène (DBO) et le potentiel méthane (BMP).

La quantité de matière organique peut être évaluée par une mesure du paramètre industriel que représente la demande biochimique (ou biologique) en oxygène. La DBO représente la quantité de dioxygène nécessaire aux micro-organismes aérobies de l'eau pour oxyder les matières organiques, dissoutes ou en suspension dans l'eau. La mesure la plus couramment réalisée est celle de la DBO₅. La DBO₅ correspond à la demande biochimique ou (biologique) en oxygène après 5 jours d'incubation de l'échantillon à une température de 20°C. Son protocole de mesure standard est décrit dans la norme NF EN 1899-1.

Le deuxième paramètre industriel utilisé est le potentiel méthane qui correspond au volume de méthane produit lors de la dégradation d'un substrat organique en présence de bactéries anaérobies. Le protocole habituel est basé sur la mesure du volume de méthane produit à partir d'une quantité connue d'un échantillon à tester en présence d'une quantité connue de micro-organismes anaérobies. L'essai se déroule dans des conditions favorables (température, pH, présence d'éléments nutritifs...) pour la dégradation de l'échantillon. Le protocole est décrit dans la norme ISO 11734. Le méthane est par la suite exploité sous forme de chaleur ou d'électricité sur le site ou injecté dans les réseaux municipaux. Le résidu de la méthanisation, le digestat, peut être valorisé sous forme de fertilisant agricole notamment.

La demande internationale WO2007/105040 concerne un dispositif d'estimation rapide de la demande biochimique en oxygène d'eau potable usée. Ce dispositif est constitué d'une membrane microbienne immobilisée attachée à une électrode, un multimètre et une station de travail portable munie d'un logiciel spécialement développé. La mesure de la DBO d'eau potable usée par le biais de ce dispositif est plus rapide, reproductible et efficace par comparaison avec les méthodes conventionnelles de type dosage.

Le brevet EP0855023B1 décrit un procédé de détermination des besoins biologiques en oxygène d'eaux usées, dans lequel un échantillon d'eaux usées est mélangé à de l'eau de dilution biologiquement neutre à un degré de dilution prédéterminé. On mesure alors, dans un bain biologique, la concentration en oxygène qui s'établit, en raison de la réaction biologique d'une biomasse prédéterminée avec l'échantillon d'eaux usées dilué dans le bain biologique. Dans ce procédé, on ajoute et mélange au bain biologique, rempli d'eau de dilution saturée en oxygène, l'échantillon d'eaux usées en quantité prédéterminée en mode de fonctionnement discontinu ; et on mesure la consommation d'oxygène qui s'établit par unité de temps et on rince le bain-biologique avec de l'eau de dilution.

Mais ces deux méthodes présentent des inconvénients, notamment car elles ne s'appliquent qu'à des échantillons liquides, et sont assez contraignantes à mettre en oeuvre.

Pour déterminer le potentiel méthane, le protocole habituel consiste à inoculer un certain nombre de flacons contenant une petite quantité de milieu à analyser avec des inoculas anaérobies en l'absence d'oxygène, souvent en atmosphère azotée. Ces flacons sont ensuite placés dans un bain d'eau ou un incubateur dont la température est contrôlée. Par analyse manuelle du volume et de la composition du gaz émis par chromatographie, le volume de méthane produit est analysé périodiquement, jusqu'à stabilisation de la courbe du volume de méthane cumulé. La durée d'analyse est de 20 à 60 jours. Ainsi, cette procédure analytique requiert un appareillage de laboratoire couteux, et est très complexe et exigeante en matière de temps et de travail. La composition en biogaz et méthane produits ne peut être analysée que ponctuellement et manuellement, rendant difficile sa mise en place de manière routinière au sein d'un laboratoire. Il existe donc un réel besoin pour un test automatisé qui fournisse des données de qualité de façon simple, fiable et rapide.

La demande internationale WO 2006/079733 propose d'utiliser la fluorescence pour évaluer l'action oxydante des bactéries sur la matière organique à dégrader. Une sonde fluorescente pénètre dans les bactéries et réagit à chaque échange d'électrons. Il s'agit donc d'une méthode directe d'appréciation de l'activité bactérienne. Cette méthode permet seulement d'évaluer le devenir d'une matière organique ou d'associations de la matière organique avec certains éléments du sol et concerne essentiellement l'analyse d'échantillons liquides en provenance du sol, mais ne fournit pas de moyen d'obtention de paramètres industriels d'usage courant, comme la DBO₅ ou le BMP.

L'article de Dudal Y. et al. (Anal. Bioanal. Chem., (2006), 384, 175-179) reprend le contenu de la demande internationale WO 2006/079733 et décrit un test de fluorescence sur microplaque pour la mesure de l'activité catabolique induite par les matières organiques dissoutes (dissolved organic matter ou DOM). Cette technique permet, à partir de courbes de calibration de glucose, de calculer la quantité minéralisable d'un DOM et de classifier les DOM en fonction de leur capacité à réagir avec des bactéries.

L'article de Christian Guyard (L'eau, l'industrie et les nuisance, (01/08/10), no 334, 51-58) est une revue sur les différentes techniques utilisées pour la mesure de DBO₅ et les problèmes inhérents à ces techniques. Il est fait référence à l'invention objet de la demande internationale WO 2006/079733 et à la corrélation qui existerait entre la mesure de fluorescence et la mesure de DBO₅ sans toutefois préciser comment elle est établie.

Un but de l'invention est de pallier les inconvénients de l'état de la technique, et en particulier d'améliorer les points suivants :
- Permettre une mesure rapide et simple à mettre en oeuvre;
- Proposer une méthode applicable à de nombreux types d'échantillons organiques;
- Améliorer la sensibilité de la méthode ;
- Etendre la gamme de concentrations mesurables par le procédé ;
- Proposer une méthode permettant l'utilisation de micro-organismes adaptés à chaque application opérationnelle ;
- Proposer une méthode permettant un travail en haut-débit ;
- Fournir des résultats sous la forme de paramètres industriels connus.

Pour cela, l'invention propose de mesurer la biodégradabilité des substrats organiques par la détection fluorescente et/ou colorimétrique de l'activité microbienne générée par l'ajout de substrats organiques dans un mélange de micro-organismes, d'en déduire par étalonnage la valeur attendue pour chaque domaine d'applications et de quantifier la matière organique présente. Cette mesure est réalisée sur un format permettant la multiplicité des mesures avec un traitement des données automatisé.

Cette invention permet de réaliser des mesures d'équivalents aux méthodes classiques de DBO₅ et de BMP, mais le procédé est utilisable également pour mesurer d'autres potentiels dans le domaine environnemental, tels qu'un potentiel fermentaire ou fertilisant, mais aussi dans les domaines de l'agroalimentaire (industrie du lait) et de la pétrochimie. Ce procédé peut également être utilisé pour réaliser le diagnostic d'un bioréacteur, mesurer les performances d'épuration ou étudier les effets inhibiteurs ou toxiques de différents produits (effluents, substrats organiques etc...). Il peut aussi permettre d'obtenir des résultats comparables à ceux de la respirométrie.

Aussi l'invention a pour objet un procédé de mesure directe de biodégradabilité d'échantillons organiques comprenant les étapes suivantes :
- préparation de l'échantillon,
- incubation, pendant une durée comprise entre 1 et 48 heures, avantageusement entre 12 et 48 heures, avantageusement entre 12 et 24 heures, au sein d'une microplaque, de l'échantillon avec un bioréactif fluorescent et/ou colorimétrique et un inoculum de micro-organismes susceptibles de dégrader ledit échantillon,
- analyse de l'absorbance - fluorescence émise par le mélange au cours du temps, ladite analyse d'absorbance - fluorescence comprenant les deux étapes suivantes :
   a) mesure d'une intensité d'absorbance - fluorescence émise suite à la dégradation de l'échantillon par l'inoculum de micro-organismes, ledit profil d'intensité de fluorescence obtenu permettant :
      - soit de déterminer le temps minimal de mesure par analyse d'un coefficient de détermination d'une courbe de calibration, ladite courbe de calibration reliant l'intensité de la fluorescence à la concentration en matière organique étant obtenue en réalisant des mesures d'absorbance - fluorescence sur des échantillons de concentrations croissantes connues constituant une gamme-étalon, et/ou par comparaison des résultats obtenus sur des échantillons de concentrations connues par une méthode habituelle « normée »,
      - soit de déduire des profils de vitesse instantanée de biodégradation,
   b) utilisation de l'intensité d'absorbance - fluorescence mesurée pour calculer une concentration de matière organique de référence, grâce à une corrélation soit par une gamme-étalon, soit par un modèle mathématique ledit procédé comprenant en outre une étape de calcul d'un paramètre industriel à partir de la concentration de matière organique calculée à l'étape b).

Conformément à l'invention, les profils de vitesse instantanée de biodégradation peuvent être calculés par toute technique connue de l'homme du métier, notamment par dérivée du profil d'intensité. Les profils de vitesse permettent de sélectionner par simple lecture la vitesse instantanée maximale qui est directement corrélée à la concentration en substrat.

Conformément à l'invention, les échantillons peuvent être des eaux usées, des eaux potables, des effluents industriels, des eaux résiduaires, des substrats organiques, notamment agro-industriels ou d'élevage, des déchets industriels, agro-industriels et agricoles ou compost et des boues de station d'épuration, des algues...

Ils peuvent être utilisés directement ou après dilution.

Dans un mode de réalisation avantageux de l'invention, la mesure de l'intensité de l'absorbance - fluorescence est réalisée par le dessous de la plaque.

Dans un autre mode avantageux de réalisation de l'invention, l'échantillon à analyser est prélevé sur un site de traitement et l'inoculum de micro-organismes provient d'un système bactérien de composition stable dans le temps présent sur ce même site.

Si nécessaire, l'inoculum est passé sur des filtres PES de maille 1,2 µm avant utilisation.

Conformément à l'invention, l'inoculum de microorganismes peut être issu du milieu de prélèvement lui-même ou être préparé à partir de souches lyophilisées ou d'une culture de souches bactériennes. Ces souches et cultures sont disponibles dans le commerce et bien connues de l'homme du métier.

Conformément à l'invention lorsque la mesure est réalisée en conditions aérobies, elle est réalisée soit en laissant la microplaque ouverte, soit en la recouvrant d'un film permettant les échanges d'oxygène sans évaporation.

Dans un mode de réalisation avantageux de l'invention, l'incubation est réalisée pendant une durée comprise entre 1 heures et 24 heures, avantageusement entre 12 et 24 heures en milieu aérobie et au moins égal à 10 heures en milieu anaérobie.

Dans un autre mode de réalisation de l'invention, les mesures sont réalisées au minimum toutes les heures et au maximum toutes les 15 minutes.

Dans un mode de réalisation avantageux de l'invention, la fluorescence mesurée est convertie en mgO₂.L⁻¹, unité de la DBO₅ (demande biologique en oxygène sur 5 jours), par comparaison avec une gamme-étalon.

Dans un autre mode de réalisation avantageux de l'invention, la fluorescence mesurée est convertie en mgO₂.L⁻¹, par utilisation d'une modèle mathématique reliant l'intensité de fluorescence à la concentration.

Dans un autre mode de réalisation avantageux de l'invention l'équation mathématique est ajustée en fonction des intensités de fluorescence mesurées sur des solutions de contrôle de concentrations connues placées dans les mêmes conditions que les échantillons à analyser.

Dans un autre mode de réalisation avantageux de l'invention la mesure est réalisée en conditions anaérobies, notamment en recouvrant chaque puits de la microplaque de paraffine puis en fermant la microplaque à l'aide d'un couvercle, la plaque pouvant être retournée pour que la mesure de fluorescence se fasse par le dessus.

Conformément à l'invention, dans le cas d'une mesure dans des conditions anaérobies, le temps de mesure choisi de façon automatisée peut être celui auquel le coefficient de détermination de la courbe de calibration est le plus proche de 1 sur une durée totale d'incubation.

Dans un mode de réalisation avantageux de l'invention en condition anaérobies, la fluorescence émise est convertie en LCH₄.Kg⁻¹ de matière brute, selon l'unité usuelle d'expression du potentiel méthane d'après un calcul comprenant les étapes suivantes :
- conversion des intensités de fluorescence en gC-Acétate.Kg⁻¹ à l'aide de l'équation mathématique issue de la régression linéaire de la courbe de calibration, ou
- référence à une base de données constituée BMP en LCH₄.Kg⁻¹ de matière brute pour prédire le potentiel méthane en LCH₄.Kg⁻¹ de matière brute.

Avantageusement, conformément à l'invention, le potentiel méthane d'échantillons inconnus est directement estimé en LCH₄.Kg⁻¹ de matière brute à partir de la courbe de calibration.

Le procédé selon l'invention peut mettre en oeuvre un kit comprenant au moins une microplaque, au moins un bioréactif fluorescent et/ou colorimétrique et des solutions-étalons et/ou de contrôle.

Conformément à l'invention, l'étape d'analyse de l'absorbance-fluorescence est mise en oeuvre par un système comprenant un lecteur de fluorescence adapté au format microplaque et des moyens de calculs agencés pour mettre en oeuvre ledit procédé.

L'invention a également pour objet un procédé de quantification de matière organique présente dans un échantillon mettant en oeuvre la mesure de biodégradabilité décrite précédemment.

Ainsi selon l'invention, la mesure comprends plusieurs étapes :
- Mise au point de la méthode : choix de l'inoculum et détermination du temps d'incubation optimal,
- Préparation de l'échantillon
- Mise en contact et incubation au sein d'une microplaque de l'échantillon avec un inoculum de micro-organismes susceptible de dégrader ledit échantillon et avec un bioréactif fluorescent et/ou colorimétrique,
- Analyse de l'absorbance et/ou fluorescence émise par le mélange au cours du temps.

Les inocula provenant de sources, de milieux ou d'environnements différents, dans des conditions de croissance différentes, ne réagissent pas de la même manière lorsqu'on les met en contact avec un même substrat. Afin de déterminer la relation entre l'activité bactérienne mesurée par l'absorbance/fluorescence et le paramètre industriel visé, une caractérisation par la méthode de référence (DBO ou BMP) doit être réalisée. Cette caractérisation est réalisée sur les solutions des gammes étalons utilisées dans la méthode de l'invention, soit des solutions synthétiques (exemple : solution de mélange glucose + acide glutamique) ou sur des échantillons réels (exemple : suspension de biodéchets broyés à différentes concentrations). Le lien entre les concentrations des solutions étalons et le paramètre industriel est ainsi défini et sera utilisée pour la quantification des échantillons à tester.

Le temps d'incubation minimal et/ou optimal est déterminé en fonction de la méthode de traitement de données utilisée.

L'analyse de l'absorbance - fluorescence se déroule en trois étapes réalisées par un système automatisé :
a) mesure de l'intensité de l'absorbance - fluorescence par un détecteur, dans un temps minimal de mesure déterminé préalablement au cours de l'étape de caractérisation de l'inoculum,
b) utilisation des profils d'intensités ou des profils de vitesses instantanées pour calculer une concentration de matière organique de référence, grâce à une corrélation soit par une gamme-étalon, soit par un modèle mathématique, cette molécule de référence pouvant être le mélange glucose - acide glutamique pour la mesure de DBO₅ et l'acétate pour la mesure du BMP, ou tout autre molécule représentant un substrat modèle pour la biodégradation étudiée ;
c) la traduction de cette valeur de concentration de matière organique de référence en un paramètre industriel, à partir de la corrélation déterminée au cours de la caractérisation de l'inoculum.

Dans certains cas, notamment en conditions anaérobies, les étapes b) et c) sont confondues.

L'algorithme de traitement des données mis en place sert à automatiser la décision du temps de lecture et l'incubation et à interpréter des résultats d'absorbance - fluorescence en unités de biodégradabilité couramment utilisées dans le milieu industriel: mgO₂.L⁻¹ pour l'équivalent DBO₅ classiquement utilisé pour évaluer la biodégradabilité des eaux usées, LCH₄.kg⁻¹ de matière brute pour l'équivalent potentiel méthane.

L'invention a donc pour objet un procédé de mesure de biodégradabilité, permettant d'obtenir de manière rapide, simple et automatisée un paramètre industriel indicatif, ledit paramètre permettant de quantifier la matière organique présente dans un échantillon.

D'autres particularités et avantages de l'invention ressortiront de la description détaillée et des dessins annexés sur lesquels :
- la FIGURE 1 représente schématiquement la mise en oeuvre du procédé selon l'invention en conditions aérobies ;
- la FIGURE 2 présente les dilutions d'échantillons « types » en fonction de la gamme utilisée pour des mesures en conditions aérobies ;
- la FIGURE 3 donne un exemple de composition du tampon de dilution à utiliser pour la préparation des échantillons.
- la FIGURE 4 représente schématiquement la mise en oeuvre du procédé selon l'invention en conditions anaérobies ;
- la FIGURE 5 donne un exemple de disposition des échantillons dans les puits d'une microplaque utilisée conformément à l'exemple 1;
- la FIGURE 6 donne un exemple de concentrations d'échantillons de gamme-étalon ;
- la FIGURE 7 est un graphe montrant l'évolution des coefficients de détermination de la courbe de calibration en fonction du temps d'incubation des échantillons-étalons, en conditions anaérobies conformément à l'exemple 1 ;
- la FIGURE 8 donne trois exemples de courbes d'étalonnage représentant les variations de l'intensité de fluorescence des échantillons de la gamme-étalon en fonction de la concentration en gC-Acétate.L⁻¹, pour trois temps d'incubation : 1 heure, 20 heures et 33 heures en conditions anaérobies conformément à l'exemple 1.

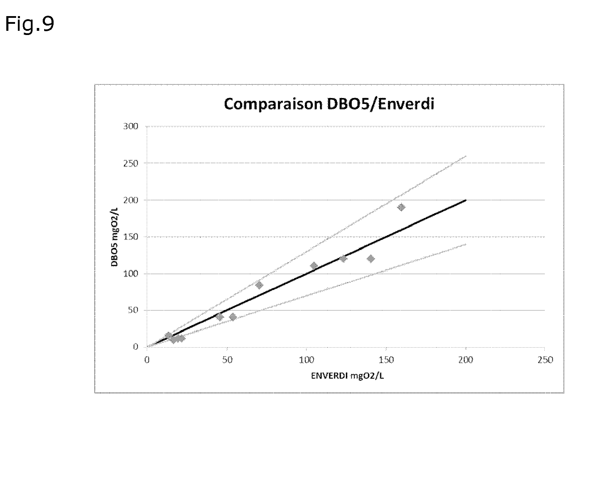

- La FIGURE 9 illustre la corrélation entre une mesure de DBO₅ classique et la mesure réalisée selon l'invention avec le kit Enverdi® conformément à l'exemple 2. La courbe noire correspond à la courbe pour laquelle la corrélation est égale à 1 (mesure Enverdi® - mesure DBO₅ normée), les courbes grises correspondent à la courbe pour laquelle la corrélation est égale à 1,3 (mesure Enverdi® = 1,3 mesure DBO₅ normée) et à la courbe pour laquelle la corrélation est égale à 0,7 (mesure Enverdi® = 0,7 mesure DBO₅ normée). (◊) représentent les mesures réalisées sur les échantillons conformément à l'exemple 2.

Le support de mesure consiste en une microplaque de 96 puits à fond transparent. La lecture se fait de préférence par en-dessous de la microplaque. L'intérêt de ce support est qu'il permet de travailler sur de très faibles volumes d'échantillons et de réactifs, et de réaliser en parallèle un grand nombre de mesures sur un ou plusieurs échantillons.

Les caractéristiques de la plaque varient en fonction du type de biodégradabilité à mesurer. Pour une biodégradabilité aérobie, la microplaque est éventuellement recouverte d'un film de culture permettant de laisser passer l'air tout en évitant l'évaporation. Pour une biodégradabilité anaérobie, chaque puits de la microplaque est recouvert de paraffine puis la microplaque est fermée à l'aide d'un couvercle. En conditions anaérobies avec des échantillons en suspension, la plaque est étanche et peut être retournée pour une lecture par le dessus afin que les matières en suspension tombent sur la paraffine et ne gênent pas la mesure.

En fonction du type de mesure à réaliser, aérobie ou anaérobie, le protocole à mettre en oeuvre est différent.

### Protocole de mesure de biodégradabilité aérobie (par exemple mesure de la DBO₅)

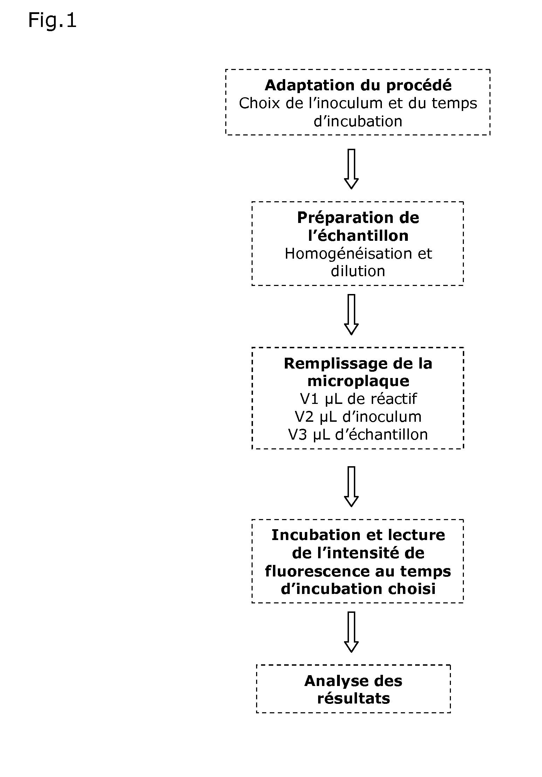

Les étapes de ce protocole sont résumées dans la FIGURE 1.

### Sélection de l'inoculum

Avant de mettre en oeuvre le procédé, il est nécessaire de sélectionner l'inoculum de micro-organismes à utiliser pour réaliser la mesure.

Pour des mesures de biodégradabilité aérobie, le choix de l'inoculum dépend :
- de la disponibilité ou non sur le site de prélèvement de l'échantillon d'un effluent chargé en bactéries, de composition assez stable dans le temps, du type eau de sortie de station d'épuration des eaux usées (STEP) ou tout autre effluent industriel chargé en bactérie ;
- de la possibilité de préparer sur place un inoculum à partir de souches lyophilisées, dépendant notamment de la disponibilité de matériel de laboratoire, et la possibilité de stockage dans les conditions requises.

Les inocula qui peuvent être utilisés sont :
- eau de sortie de station STEP sans traitement tertiaire de désinfection ;
- eau d'entrée de STEP diluée entre 10 et 100 fois ;
- inoculum spécifique DBO₅ en pastilles ou gélules lyophilisées ;
- suspension de biofilms ;
- autre mélange de bactéries ou souches de bactéries adaptées à l'application.

Le large choix d'inocula utilisables pour mettre en oeuvre le procédé selon l'invention permet une adaptation du procédé à de multiples applications sur le terrain ou en laboratoire. L'homme du métier saura choisir, à la lumière de ses connaissances l'inoculum adapté.

### Caractérisation de l'inoculum

Une phase préalable d'étude et de caractérisation de l'inoculum est nécessaire afin de déterminer le lien entre l'évolution de l'intensité d'absorbance-fluorescence et le paramètre industriel que l'on souhaite exprimer. Cette étape consiste à analyser en parallèle dans les conditions opératoires de l'invention et selon la méthode de référence avec le même inoculum :
- soit des solutions synthétiques contenant des concentrations croissantes d'un substrat (par exemple un mélange glucose-acide glutamique, de la cellulose, de l'acétate de sodium...)
- soit des préparations d'un échantillon à différentes dilutions
- soit des préparations de plusieurs échantillons de matrices différentes

La comparaison des profils d'intensités ou des profils de vitesse d'évolution de fluorescence et la mesure de DBO₅ serviront à relier la gamme étalon au paramètre industriel, et donc à la quantification des échantillons analysés selon la méthode de l'invention, pour cet inoculum.

### Sélection du temps d'incubation

Après le choix d'un ou plusieurs inocula, une deuxième phase d'adaptation de la mise en oeuvre du procédé selon l'invention consiste en la détermination du temps d'incubation.

Deux types d'analyses sont réalisés en parallèle sur des échantillons réels et des solutions de contrôle synthétiques en concentrations croissantes correspondant à la gamme de calibration :
- des mesures selon le procédé de l'invention ; dans ce cas, une analyse est réalisée toutes les 30 minutes, sur une durée totale d'incubation de 24h.
- des mesures du paramètre industriel, comme par exemple une mesure DBO₅ selon la norme.

Ensuite, une comparaison est réalisée entre les résultats obtenus pour chaque type de mesure. Pour chaque temps d'incubation de la méthode selon l'invention, la qualité de la courbe de calibration, c'est-à-dire soit la valeur du coefficient de détermination de la courbe de calibration, soit les vitesses instantanées maximales mesurées, et les concentrations calculées des échantillons et des points de contrôle sont relevées. La comparaison des résultats obtenus pour chaque temps d'incubation avec la méthode de référence permet de déterminer le temps d'incubation auquel on a à la fois une bonne corrélation de la gamme étalon et une bonne prédiction du paramètre industriel des échantillons et points de contrôles. Par exemple, le temps d'incubation choisi est celui auquel le coefficient de détermination de la courbe de calibration est égal à 0,98 et l'erreur standard de prédiction entre une valeur de potentiel mesurée selon une méthode normée et une valeur mesurée selon le procédé est inférieure à 30%.

Si plusieurs inocula ont été testés, celui qui donne les meilleurs résultats dans le temps d'incubation le plus court est sélectionné.

### Protocole de préparation de l'échantillon

Les échantillons à analyser subissent une simple homogénéisation avant prélèvement, et des dilutions en fonction de leur concentration et des gammes utilisées.

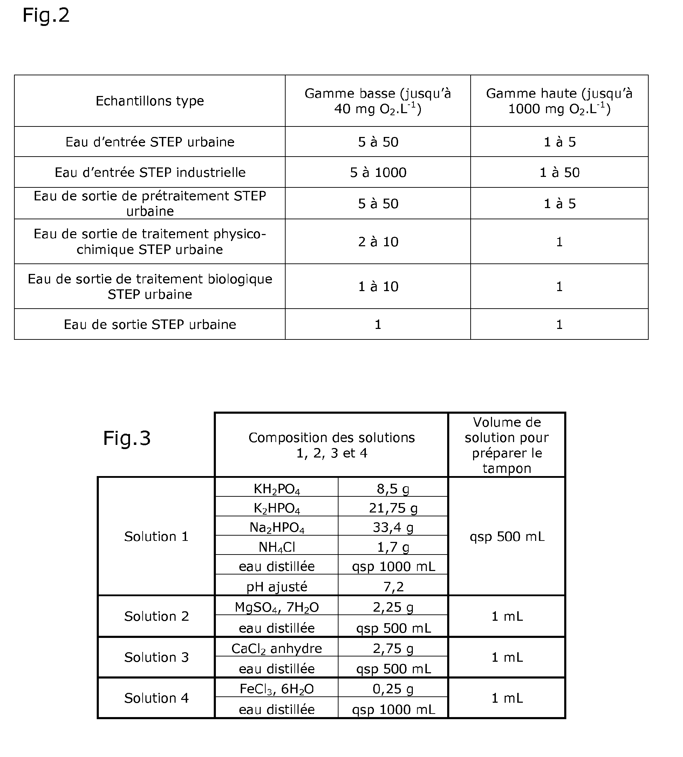

Le tableau présenté dans la FIGURE 2 présente les dilutions des échantillons « type » en fonction de la gamme utilisée, à titre d'information. D'autres gammes peuvent être développées en fonction des besoins des utilisateurs.

L'échantillon est dilué dans de l'eau distillée ou dans un tampon dont un exemple de composition est donné dans le tableau de la FIGURE 3.

### Protocole de remplissage de la microplaque

Les puits d'une microplaque à fond transparent sont remplis selon le protocole suivant :
- V₁ µL de réactif
- V₂ µL d'inoculum
- V₃ µL d'échantillon, de solution standard ou de solution de contrôle.

L'optimisation des volumes et concentrations de réactifs permet d'augmenter la sensibilité de la méthode, de diminuer le temps de lecture et d'incubation et d'améliorer la correspondance avec les méthodes classiques.

### Réactif

Le réactif est une solution d'un bioréactif fluorescent et/ou colorimétrique indicateur de prolifération microbienne dilué dans un tampon.

Une sonde fluorescente utilisée est par exemple un révélateur rédox fluorescent sensible au catabolisme de la matière organique de l'échantillon. Le bioréactif fluorescent peut être choisi en particulier parmi le groupe constitué des compositions commerciales de dérivés de resazurine (commercialisée notamment sous les produits Alamarblue™, voir brevet U.S. 5,501,959, ou MTT, ou PrestoBlue™). L'Alamarblue™ présente le double avantage du changement de couleur mais aussi de la formation d'un produit fluorescent. Le changement de couleur entre la forme oxydée non fluorescente, bleue et la forme réduite fluorescente, violette est marqué et s'observe à l'oeil nu. Le réactif fluorescent choisi retenu pour sa sensibilité et pour sa capacité à révéler tous les métabolismes (aérobie ou anaérobie).

Le tampon de dilution est choisi pour ne pas interférer avec la mesure. Il peut être préparé selon le protocole présenté dans le tableau de la FIGURE 3.

Le taux de dilution du bioréactif fluorescent et/ou colorimétrique dans le tampon est défini en fonction des applications et des gammes de mesure.

Selon un mode de réalisation particulier du procédé selon l'invention, le pH de la solution 1 peut être ajusté, dans une gamme variant entre 6,5 et 8,5.

Selon un autre mode de réalisation selon l'invention, le bioréactif n'est dilué que dans la solution 1 (FIGURE 3).

Le volume V₁ de réactif 1 dilué est compris entre 10 et 180 µL, avantageusement compris entre 50 et 150 µL et de préférence égal à 90 µL.

### Inoculum

L'inoculum, sélectionné dans la première étape, peut être un échantillon « réel », par exemple, une eau d'entrée ou de sortie de STEP, dilué ou non, à l'eau distillée ou dans un tampon tel que celui décrit dans le tableau de la FIGURE 3. Il peut également s'agir d'un inoculum « synthétique », sous forme de pastilles ou gélules de bactéries lyophilisées.

Le volume d'inoculum V₂ est compris entre 10 et 180 µL, avantageusement compris entre 50 et 150 µL et de préférence égal à 90 µL.

### Echantillonlstandard/solution de contrôle

Le volume de la solution à analyser qui est soit un échantillon dilué ou non, soit un point de la gamme étalon, soit une solution de contrôle, est compris entre 10 µL et 180 µL, avantageusement compris entre 50 et 150 µL et de préférence égal à 90 µL.

### Incubation

L'incubation se fait à une température adaptée en fonction du potentiel à mesurer. Pour des mesures de DBO₅, l'incubation est réalisée à une température comprise entre 29°C et 31°C, de préférence égale à 30°C. La durée totale d'incubation est comprise entre 1 heure et 24 heures, avec des mesures d'absorbance - fluorescence à une fréquence définie. La fréquence des mesures est au maximum toutes les 15 minutes et au minimum toutes les heures. L'incubation se fait directement dans le lecteur qui contrôle les conditions de température et d'agitation.

Ce protocole permet d'acquérir des lectures d'absorbance - fluorescence selon un pas de temps très régulier, de manière automatisée afin de suivre en continu la biodégradation.

### Analyse des résultats selon la méthode 1

L'analyse des résultats se déroule en trois étapes :

### Etape 1 : Elaboration de la méthode de calcul entre intensité de fluorescence et concentration en mgO₂.L⁻¹

On peut distinguer trois cas :
- Cas 1 : Une mesure de gamme étalon complète est réalisée en parallèle avec les mesures des échantillons de concentrations inconnues à analyser pour obtenir une courbe de calibration. Pour chaque échantillon, l'équation de la courbe de calibration représentant l'intensité de la fluorescence en fonction de la concentration en mgO₂.L⁻¹ est utilisée pour calculer les concentrations des échantillons analysés. Cette courbe de calibration est obtenue en définissant au préalable une relation de proportionnalité entre une concentration en glucose-acide glutamique et une valeur de DBO₅ en mgO₂.L⁻¹ par analyse de solutions synthétiques de glucose-acide glutamique selon la méthode normée DBO₅.
- Cas 2 : Seuls les échantillons de concentrations inconnues sont analysés et un modèle mathématique interne est utilisé pour calculer leurs concentrations. L'équation du modèle interne reliant l'intensité de fluorescence à la concentration en mgO₂.L⁻¹ est déterminée par l'exploitation de résultats de nombreuses expériences préalables, au minimum au nombre de quatre. Pour chaque échantillon, l'équation du modèle interne est utilisée pour déterminer les concentrations des échantillons. Des solutions de contrôle de concentrations connues peuvent éventuellement être analysées en parallèle pour vérifier la validité du modèle.
- Cas 3 : Les échantillons de concentrations inconnues ainsi que des solutions de contrôle de concentrations connues sont analysés. Les mesures d'intensité de fluorescence sur les solutions de contrôle sont utilisées comme moyens de comparaison pour ajuster l'équation du modèle mathématique utilisé dans le cas 2 précédemment cité. La nouvelle équation obtenue est utilisée pour déterminer les concentrations des échantillons analysés.

Le facteur de dilution des échantillons est pris en compte dans le calcul des concentrations.

### Etape 2 : Vérification du temps d'incubation

Il est utile de vérifier, si possible, que les conditions déterminées au préalable sont bien adaptées à l'expérience, en particulier lorsque l'inoculum choisi est un inoculum « réel », issu d'un prélèvement. Des variations sur le process de la STEP ou des perturbations climatiques peuvent induire des modifications dans la quantité et qualité des bactéries présentes dans le prélèvement, d'où des conditions d'incubation différentes des conditions choisies dans un premier temps.

La vérification du temps d'incubation peut se faire selon l'une des méthodes suivantes :
- Suivi du coefficient de détermination R² de la courbe de calibration au cours du temps : la variation du R² au cours du temps est comparée avec celle obtenue pendant les premières expériences permettant de déterminer le temps d'incubation. La valeur du R² au temps d'incubation choisi est également relevée. Sa valeur doit être supérieure à 0,98. Cette méthode est valable si une gamme-étalon est passée en plus des échantillons sur la plaque.
- Vérification de l'intensité de fluorescence des solutions de contrôle : les valeurs de l'intensité de fluorescence des solutions de contrôle sont comparées avec les valeurs attendues au temps d'incubation choisi. L'écart observé doit être inférieur à 10%. Cette méthode est valable si une ou plusieurs solutions de contrôle ont été passées.
- Vérification des concentrations des solutions de contrôle : à partir de l'équation définie (calibration ou modèle interne), les concentrations des solutions de contrôle sont calculées pour chaque temps d'incubation. Les concentrations des solutions de contrôles, calculées au temps d'incubation sont comparées avec les concentrations réelles attendues. L'écart observé doit être inférieur à 10%. Cette méthode est valable si une ou plusieurs solutions de contrôle ont été passées.

Si ces vérifications amènent à la conclusion que le temps d'incubation défini au préalable n'est pas le temps optimal de cette expérience, un autre temps d'incubation doit être choisi pour la lecture des concentrations. Ce choix arbitraire est établi à partir des observations réalisées sur la courbe de calibration, notamment concernant l'évolution du R² au cours du temps, ainsi que sur l'évolution des intensités de fluorescence et/ou des concentrations calculées au cours du temps des solutions de contrôle.

### Etape 3 : Détermination des concentrations des échantillons

Après lecture de l'intensité de fluorescence pour chaque échantillon au temps d'incubation choisi, la concentration en un paramètre industriel, en particulier en mgO₂.L⁻¹ pour une mesure de DBO₅, est calculée d'après la méthode de détermination décrite dans l'étape 1.

### Analyse des résultats selon la méthode 2

L'analyse des résultats se déroule en 2 étapes :

### Etape 1 : Elaboration de la méthode de calcul entre intensité de fluorescence et concentration en mgO₂/L.

On peut distinguer 3 cas :
- Cas 1 : Une mesure de gamme étalon complète est réalisée en parallèle avec les mesures des échantillons de concentrations inconnues à analyser pour obtenir une courbe de calibration. La courbe de calibration relie la vitesse maximale d'évolution de fluorescence au cours de l'analyse à la concentration de la solution étalon, qui peut être exprimée directement avec l'unité du paramètre industriel, selon les résultats obtenus lors de la caractérisation de l'inoculum. Pour les échantillons, la vitesse maximale d'évolution de fluorescence est déterminée après un temps d'adaptation des bactéries de l'échantillon au nouveau milieu (température, nutriments, pH...) qui peut être de 1h à 5h.
- Cas 2 : Seuls les échantillons de concentrations inconnues sont analysés et un modèle mathématique interne est utilisé pour calculer leurs concentrations. L'équation du modèle interne reliant la vitesse maximum d'évolution de fluorescence à la teneur en mgO₂/L est déterminée par l'exploitation des résultats de nombreuses expériences préalables, au minimum au nombre de quatre. Pour chaque échantillon, l'équation du modèle interne est utilisée pour déterminer les concentrations des échantillons. Des solutions de contrôle de concentrations connues peuvent éventuellement être analysées en parallèle pour vérifier la validité du modèle.
- Cas 3 : Les échantillons de concentrations inconnues ainsi que des solutions de contrôle de concentrations connues sont analysés. Les mesures d'intensité de fluorescence sur les solutions de contrôle sont utilisées comme moyens de comparaison pour ajuster l'équation du modèle mathématique utilisé dans le cas 2 précédemment cité. La nouvelle équation obtenue est utilisée pour déterminer les concentrations des échantillons analysés.

### Etape 2 : Détermination des concentrations des échantillons

Après lecture de l'intensité de fluorescence pour chaque échantillon et traitement des données pour déterminer la vitesse maximale d'évolution de la fluorescence au cours de l'analyse, la concentration en un paramètre industriel, en particulier en mgO₂/L pour une mesure de DBO₅, est calculée d'après la méthode de détermination décrite dans l'étape 1.

### Protocole de mesure de biodégradabilité anaérobie (par exemple, mesure du BMP)

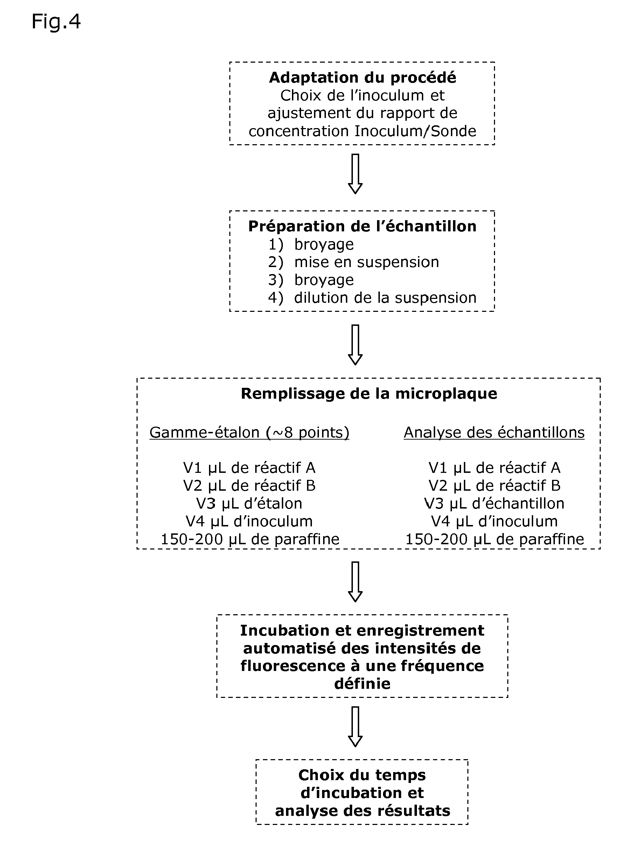

Ce protocole est résumé dans la FIGURE 4.

### Sélection de l'inoculum

Pour des mesures de biodégradabilité anaérobie, le choix de l'inoculum dépend :
- de la disponibilité ou non sur le site de prélèvement des échantillons de boues de digesteurs ou d'un système bactérien anaérobie de composition assez stable dans le temps;
- de la possibilité de préparer un inoculum modèle à partir de cultures de souches, d'un cocktail de souches, d'un digesteur de laboratoire, ou de souches lyophilisées.

Les inocula qui peuvent être utilisés sont :
- boues de digesteurs
- toute biomasse provenant d'un système anaérobie
- suspensions de biofilms anaérobies et/ou biofilms anaérobies
- cultures de bactéries, ou bactéries lyophilisées
- autre mélange de bactéries ou souches de bactéries adaptées à l'application.

Après avoir choisi l'inoculum, il est important d'ajuster le rapport de concentration Bactéries / Bioréactif fluorescent. Cette démarche s'effectue de manière empirique en effectuant le test pour différentes concentrations et/ou volumes d'inoculum et différents essais de concentrations sur le bioréactif. Ces tests sont menés sur les points étalons, nous permettant ainsi d'ajuster le rapport pour obtenir des intensités d'absorbance - fluorescence valides. Le volume total dans un puits ne peut pas dépasser 300µL.

Selon un mode de réalisation particulier du procédé selon l'invention, des mesures peuvent être réalisées en parallèle sur des échantillons au BMP connus pour vérifier l'exactitude des résultats obtenus selon l'invention.

### Caractérisation de l'inoculum

Une phase préalable d'étude et de caractérisation de l'inoculum est nécessaire afin de déterminer le lien entre l'évolution de l'intensité d'absorbance-fluorescence et le paramètre industriel BMP que l'on souhaite exprimer. Cette étape consiste à analyser en parallèle dans les conditions opératoires de l'invention et selon la méthode de référence BMP, avec le même inoculum :
- soit des solutions synthétiques contenant des concentrations croissantes d'un substrat (par exemple l'acétate de sodium, la cellulose...)
- soit des préparations d'un échantillon à différentes dilutions (exemple : une suspension d'un prélèvement de biodéchets ou de boues de station d'épuration à différentes concentrations)
- soit des préparations de plusieurs échantillons de matrices différentes (ex : des biodéchets, des déchets agroalimentaires, des boues de station d'épuration, des lisiers...).

La comparaison des profils d'intensités ou des profils de vitesse d'évolution de fluorescence et des paramètres industriels obtenus par la méthode de référence serviront à relier les gammes étalons aux paramètres industriels, et donc à la quantification des échantillons analysés selon la méthode de l'invention, pour cet inoculum.

### Protocole de préparation de l'échantillon

Le procédé selon l'invention permet l'analyse d'échantillons à l'état liquide et d'échantillons à l'état solide.

Les échantillons à analyser sont prélevés de telle manière que l'échantillon soit représentatif de l'ensemble du gisement de matières.

Chaque échantillon est ensuite mixé à l'aide d'un mixeur alimentaire.

L'échantillon subit ensuite une mise en suspension. Par exemple, 20g d'échantillon solide sont mis en suspension dans de l'eau distillée jusqu'à atteindre 200g. Cette masse est adaptée selon la facilité de mise en suspension, la disponibilité ou l'homogénéité du produit.

Si la volonté est de comparer le résultat des analyses selon le procédé de l'invention avec des BMP, à cette étape il est souhaitable de mesurer la densité des échantillons, si l'état de l'échantillon le permet.

La suspension obtenue est à nouveau homogénéisée par broyage, avec un mixeur alimentaire.

Cette suspension ou l'effluent brut peuvent ensuite être dilués sous 5 concentrations : 1/50, 1/100, 1/200, 1/250, 1/500, dilutions habituellement pratiquées.

La mesure peut ainsi être conduite sur l'échantillon brut ou la suspension « mère », ainsi que sur l'ensemble ou une sélection de solutions diluées.

### Protocole de remplissage de la microplaque

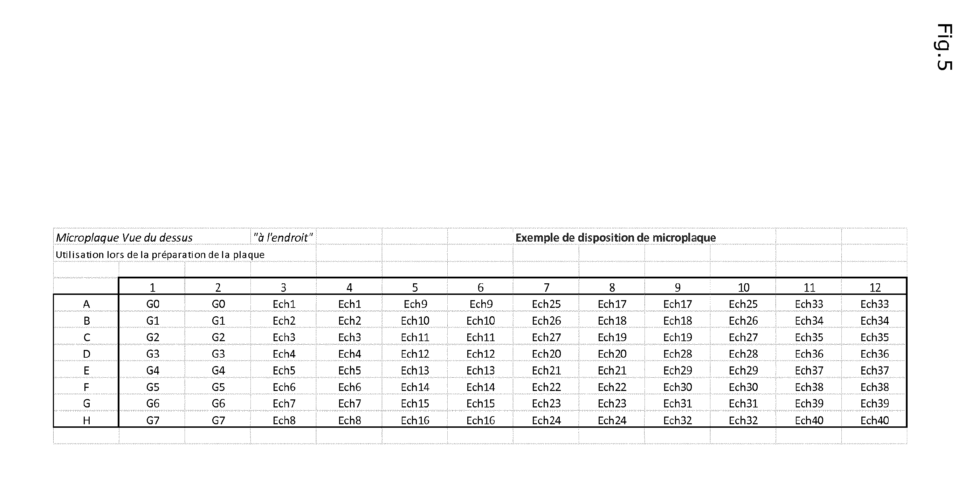

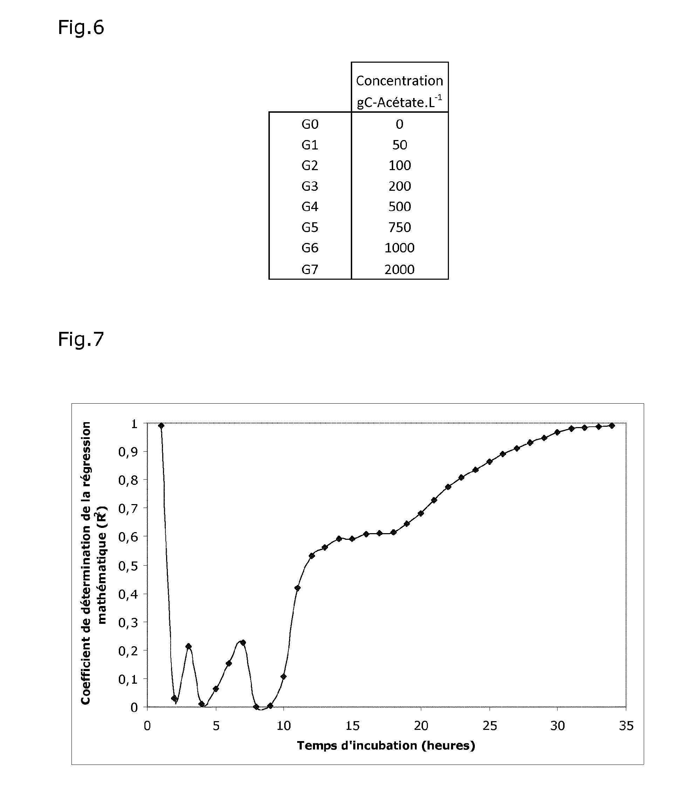

La FIGURE 5 donne un exemple de disposition de microplaque en conditions anaérobies. Les solutions étalons G0 à G7 correspondent à différentes concentrations croissantes de la gamme-étalon, dont des exemples de valeurs sont données dans la FIGURE 6. Le schéma d'organisation de la microplaque est renseigné au programme du lecteur d'absorbance - fluorescence, en termes d'identification des échantillons.

Les puits d'une microplaque à fond transparent sont remplis selon le protocole suivant :
- V₁ µL de réactif A
- V₂ µL de réactif B
- V₃ µL d'échantillon, de solution étalon ou de solution de contrôle
- V₄ µL d'inoculum
- 150-200 µL de paraffine

### Réactif A

Le réactif A est une solution tampon dont un exemple de composition est donné dans le tableau de la FIGURE 3. Dans cet exemple, le pH de la solution 1 est ajusté à la valeur de 7,2.

Selon un mode de réalisation particulier du procédé selon l'invention, le pH de la solution 1 peut être aussi ajusté, dans une plage variant par exemple entre 6,5 et 8,5, ou dans une autre plage de pH adaptée à l'application

Selon un autre mode de réalisation du procédé selon l'invention, le bioréactif n'est dilué que dans la solution 1.

### Réactif B

Le réactif B est un bioréactif fluorescent et/ou colorimétrique indicateur de prolifération microbienne, dilué ou non dans un tampon.

Le taux de dilution du réactif B dans un tampon est compris entre 1 et 10. Par exemple, pour des questions de sensibilité, le réactif B peut être utilisé à la concentration de commercialisation avec un volume de 100 µL.

Selon un mode de réalisation particulier du procédé selon l'invention, le volume de produit pur peut être légèrement augmenté, ou le produit peut être dilué, de 50µL à 150µL.

### Echantillon brut dilué ou non/standard/solution de contrôle

Dans chaque puits, un volume de 100 µL d'échantillon est introduit.

### Inoculum

Après prélèvement, l'inoculum est filtré au travers de filtres PES (polyethersulfone) de maille 1,2 µm, dans le but de retenir les plus grosses particules de matières organiques tout en laissant passer les cellules bactériennes constituant l'inoculum.

Selon un mode de réalisation particulier du procédé selon l'invention, dans un milieu de culture concentré ou stressé, une maille de filtration plus fine peut être utilisée. Ce choix peut être un moyen de sélection de communautés bactériennes ou de souches bactériennes favorables à l'analyse ou à la prédiction.

Selon un autre mode de réalisation, si l'inoculum est faiblement chargé en matières organiques, la filtration peut être évitée.

Suite à cette première phase de filtration, l'inoculum est introduit dilué ou non dans un puits, en respectant un volume réactionnel total compris entre 280 µL et 300 µL.

Par exemple, un inoculum issu d'un digesteur de traitement de co-produits de l'épuration de l'eau, est introduit dans le puits dans un volume de 30 µL.

### Paraffine

Afin que la mesure puisse se dérouler dans des conditions anaérobies, une goutte de paraffine est déposée sur le liquide réactionnel de chaque puits.

Selon un mode de réalisation préféré du procédé selon l'invention, un volume de 150 µL à 200 µL de paraffine est déposé à la surface de chaque puits.

### Incubation

Pendant toute la durée d'incubation, les intensités de fluorescence sont collectées de manière automatisée à une fréquence déterminée, par exemple toutes les heures. A chaque temps de mesure, l'intensité de fluorescence des points étalons permet de tracer une courbe d'étalonnage représentant la corrélation entre intensité de fluorescence et concentration acétate des points étalons ou intensité de fluorescence et BMP des points étalons. L'incubation se fait directement dans le lecteur qui contrôle les conditions de température et d'agitation.

### Analyse des résultats

### Etape 1 : Sélection du temps d'incubation

En conditions anaérobies, la sélection du temps d'incubation est réalisée de manière automatisée, en même temps que la mesure des échantillons, grâce à l'algorithme de traitement des données.

Le paramètre suivi pour la sélection du temps d'analyse des intensités de fluorescence est le coefficient de détermination R² de la courbe de calibration correspondant les solutions étalons G0 à G7, obtenue pour chaque temps de mesure.

A la fin de l'incubation, le temps d'analyse des données est choisi pour la courbe d'étalonnage présentant le meilleur coefficient de détermination. Une vérification des cinétiques d'intensité de fluorescence au cours du temps pour chaque échantillon ou étalon est effectuée et permet d'ôter des valeurs indésirables. Par exemple, une diminution de l'intensité de fluorescence, une valeur de l'intensité de fluorescence d'un échantillon supérieure à la valeur de l'intensité de fluorescence du plus haut point de la gamme-étalon, peut refléter des intensités de fluorescence associés à un métabolisme fermentaire et non méthanogène.

### Etape 2 : Méthode de calcul du potentiel méthane

Une fois que le temps d'analyse a été sélectionné, les intensités de fluorescence des échantillons de concentrations inconnues sont converties en gC-Acétate.Kg⁻¹ à l'aide de la courbe d'étalonnage.

Puis on se réfère à une base de données constituée d'échantillons ayant des valeurs connues en gC-Acétate.Kg⁻¹ et en BMP LCH₄.Kg⁻¹ de matière brute. Cette base de données permet d'effectuer la prédiction pour des échantillons dont le potentiel méthane est à prédire en LCH₄.Kg⁻¹ de matière brute. Des contrôles sur des échantillons aux BMP connus peuvent être réalisés en parallèle pour vérification.

Selon un mode de réalisation particulier du procédé selon l'invention, le potentiel méthane des échantillons inconnus est directement estimé à partir de la courbe de calibration et exprimé en LCH₄.Kg⁻¹ de matière brute. Ceci est possible dans le cas où les étalons seuls permettent une bonne prédiction du potentiel méthane d'échantillons complexes, c'est-à-dire que chaque point étalon est caractérisé par un potentiel méthane.

D'autres particularités et avantages de l'invention apparaîtront à la lecture de la description détaillée de l'exemple ci-après.

### Exemple 1: Prédiction du potentiel méthane en conditions anaérobies dans des boues de digesteur

Dans cet exemple, l'inoculum est constitué de boues d'un digesteur « infiniment mélangé » d'une station d'épuration.

La première étape consiste à prélever l'inoculum et les échantillons.

Les boues du digesteur sont prélevées à mi-hauteur d'un réacteur « infiniment mélangé ». Les échantillons intrants sont également prélevés.

L'inoculum est stocké en incubateur à 35°C, 55°C ou à la température du digesteur.

Les échantillons sont ensuite préparés d'après le protocole de mesure de biodégradabilité anaérobie décrit dans l'invention.

Les échantillons solides ne pouvant être prélevés avec une propipette de 5mL subissent les étapes de préparation suivantes :
- broyage
- mise en suspension
- broyage
- dilution

Les échantillons dit liquides pouvant être prélevés avec une propipette de 5 mL subissent seulement deux étapes de préparation :
- broyage
- dilution

La densité des échantillons est alors mesurée.

Les étapes suivantes sont le remplissage de la microplaque et la préparation de l'inoculum.

Le remplissage de la microplaque est défini par le manipulateur qui dispose les solutions étalons et les échantillons selon une disposition personnelle. Cette disposition est ensuite renseignée au programme du lecteur de fluorescence. Un exemple de disposition est schématisé dans la FIGURE 5, G0 à G7 représentant les solutions de la gamme-étalon.

Dans chaque puits de la microplaque à fond transparent correspondant à une analyse, on dispose 50 µL de tampon. Si les 96 puits sont utilisés alors on distribue le tampon dans l'ensemble des puits. Si seuls quelques puits sont nécessaires, alors on ne distribue que dans ces puits, les puits restants pouvant être utilisés pour de prochaines analyses.

Dans chacun des puits sélectionnés sont introduits 100 µL de réactif B.

Ensuite, selon la disposition convenue préalablement, 100 µL de chacune des solutions étalons G0 à G7 fournies sont disposés dans les puits correspondants, et 100 µL d'échantillon (brut, dilué ou non) sont distribués dans chacun des puits correspondants au plan de la microplaque.

A l'aide d'une seringue et d'un filtre seringue, l'inoculum est passé sur un filtre de 1,2 µm. Ensuite 30 µL de cet inoculum filtré sont distribués dans chacun des puits afin de compléter le mélange réactionnel.

Afin d'homogénéiser le mélange et de faire glisser des gouttes auparavant retenues sur les bords des puits, la microplaque est tapotée manuellement contre le plan de manipulation.

La paraffine est mise à fondre, puis prélevée à l'aide d'une propipette d'une contenance de 1mL. Un volume de 150 à 200 µL de paraffine est déposé à la surface de la solution réactionnelle dans chacun des puits. La microplaque est ensuite fermée à l'aide d'un couvercle pour assurer des conditions anaérobies.

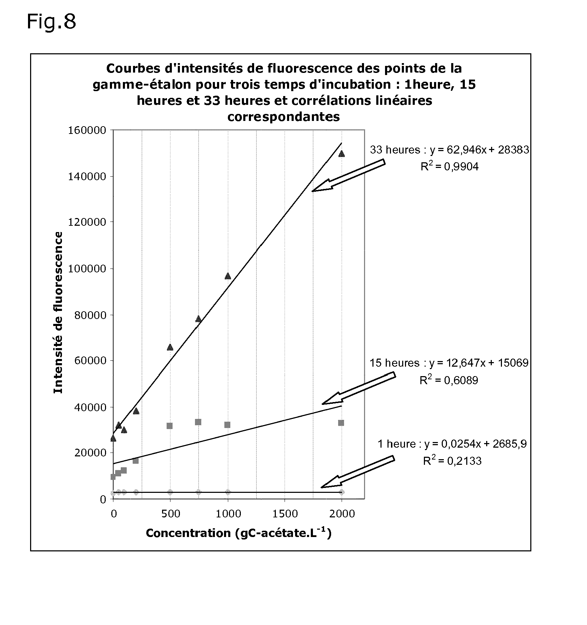

La microplaque ainsi remplie est déposée dans le lecteur pour incubation et mesure. Les intensités de fluorescence sont collectées de manière automatisée toutes les heures. A chaque collecte, l'intensité de fluorescence des points étalons G0 à G7 permet de tracer une courbe d'étalonnage représentant la corrélation linéaire entre l'intensité de fluorescence et la concentration en acétate des points étalons. Le coefficient de détermination de la corrélation linéaire permet de connaître l'adéquation entre les données observées et le modèle. La FIGURE 7 montre l'évolution des coefficients de détermination de la courbe de calibration en fonction du temps d'incubation des échantillons-étalons. Trois exemples de courbes d'étalonnage représentant les variations de l'intensité de fluorescence des échantillons de la gamme-étalon en fonction de la concentration en gC-Acétate.L⁻¹, sont donnés dans la FIGURE 8 pour des temps d'incubation de 1 heure, 20 heures et 33 heures. On observe que le coefficient de détermination R² de la courbe de calibration s'améliore au cours du temps. Pour un temps d'incubation égal à 33 heures, R² est égal à 0,99.

Il est donc choisi de manière automatisée un temps d'incubation de 33 heures pour analyser les intensités de fluorescence des échantillons.

Ensuite, les intensités de fluorescence des échantillons de concentrations inconnues sont converties en gC-Acétate.L⁻¹ à l'aide de l'équation de la courbe d'étalonnage (FIGURE 8). Puis on se réfère à une base de données constituée d'échantillons avec valeur connue en gC-Acétate.L⁻¹ et BMP LCH₄.L⁻¹ de matière brute. Cette base de données permet d'effectuer la prédiction pour des échantillons dont le potentiel méthane est à prédire en LCH₄.L⁻¹ de matière brute.

Bien sûr, l'invention n'est pas limitée à l'exemple qui vient d'être décrit et de nombreux aménagements peuvent être apportés à cet exemple sans sortir du cadre de l'invention.

La méthode selon l'invention permet d'obtenir des équivalents DBO₅ et des équivalents BMP respectivement en moins de 24 heures et en moins de 35 heures avec une excellente corrélation par rapport aux méthodes classiques de mesure de ces deux paramètres industriels.

### Exemple 2 : Prédiction de DBO₅ dans des échantillons issus d'une station d'épuration urbaine

Les échantillons sont des prélèvements réalisés tout au long du process dans une station d'épuration urbaine.

La durée de l'incubation en présence du bioréactif fluorescent est de 15h à 30 °C sur une plaque recouverte d'un film aérobie ;

L'eau d'entrée de la station d'épuration diluée 25 fois est utilisée comme type d'inoculum (microorganismes)

Le mélange d'incubation comprend 90µL de réactif comprenant la sonde fluorescente, 90µL d'inoculum et 90µL d'échantillon à tester.

La référence se fait par rapport à une gamme glucose/acide glutamique de 0 à 250mg/L.

La lecture est faite par le dessous dans un spectrophotomètre aux longueurs d'ondes suivantes : excitation 540nm ; émission 600nm.

Parallèlement la DBO₅ des échantillons est mesurée par une méthode normée classique.

Les résultats sont donnés dans la figure 9.

Toutes les mesures sont comprises entre - 30 et + 30 % par rapport à la droite pour laquelle le coefficient de corrélation est égal à 1.

La mesure selon l'invention permet donc d'avoir une très bonne évaluation de la quantité de matière organique présente très rapidement (15 heures).

## Revendications

1. Procédé de mesure directe de biodégradabilité d'échantillons organiques comprenant les étapes suivantes :
- préparation de l'échantillon,
- incubation, pendant une durée comprise entre 1 et 48 heures, avantageusement entre 12 et 24 heures, au sein d'une microplaque, de l'échantillon avec un bioréactif fluorescent et/ou colorimétrique et un inoculum de micro-organismes susceptibles de dégrader ledit échantillon, lesdits micro-organismes étant éventuellement préparés à partir de souches lyophilisées ou d'une culture de souches bactériennes,
- analyse de l'absorbance - fluorescence émise par le mélange au cours du temps, ladite analyse d'absorbance - fluorescence comprenant les deux étapes suivantes :
a) mesure d'une intensité d'absorbance - fluorescence émise suite à la dégradation de l'échantillon par l'inoculum de micro-organismes, ledit profil d'intensité de fluorescence obtenu permettant :
- soit de déterminer le temps minimal de mesure par analyse d'un coefficient de détermination d'une courbe de calibration, ladite courbe de calibration reliant l'intensité de la fluorescence à la concentration en matière organique étant obtenue en réalisant des mesures d'absorbance - fluorescence sur des échantillons de concentrations croissantes connues constituant une gamme-étalon, et/ou par comparaison des résultats obtenus sur des échantillons de concentrations connues par une méthode habituelle « normée »,
- soit de déduire des profils de vitesse instantanée de biodégradation,
b) utilisation de l'intensité d'absorbance - fluorescence mesurée pour calculer une concentration de matière organique de référence, grâce à une corrélation soit par une gamme-étalon, soit par un modèle mathématique, ledit procédé comprenant en outre une étape de calcul d'un paramètre industriel à partir de la concentration de matière organique calculée à l'étape b).

2. Procédé selon la revendication 1 **caractérisé en ce que** la mesure de l'intensité de l'absorbance - fluorescence est réalisée par le dessous de la plaque.

3. Procédé selon l'une quelconque des revendications précédentes **caractérisé en ce que** l'échantillon à analyser est prélevé sur un site de traitement et que l'inoculum de micro-organismes provient d'un système bactérien de composition stable dans le temps présent sur ce même site, ledit inoculum étant éventuellement passé sur des filtres PES de maille 1,2 µm.

4. Procédé selon l'une quelconque des revendications précédentes **caractérisé en ce que** la mesure est réalisée, avantageusement au minimum toutes les heures et au maximum toutes les 15 minutes, en conditions aérobies, soit en laissant la microplaque ouverte, soit en la recouvrant d'un film permettant les échanges d'oxygène sans évaporation.

5. Procédé selon l'une quelconque des revendications précédentes **caractérisé en ce que** la fluorescence mesurée est convertie en mgO₂.L⁻¹, unité d'expression de la DBO₅ selon la norme par comparaison avec une gamme-étalon ou par utilisation d'un modèle mathématique reliant l'intensité de fluorescence à la concentration.

6. Procédé selon la revendication 5 **caractérisé en ce que** l'équation mathématique est ajustée en fonction des intensités de fluorescence mesurées sur des solutions de contrôle de concentrations connues placées dans les mêmes conditions que les échantillons à analyser.

7. Procédé selon l'une quelconque des revendications 1 à 4 **caractérisé en ce que** la mesure est réalisée en conditions anaérobies, notamment en recouvrant chaque puits de la microplaque de paraffine puis en fermant la microplaque à l'aide d'un couvercle, la plaque étant éventuellement retournée pour que la mesure de fluorescence se fasse par le dessus.

8. Procédé selon la revendication 7 **caractérisé en ce que** le temps de mesure choisi de façon automatisée est celui auquel le coefficient de détermination de la courbe de calibration est le plus proche de 1 sur une durée totale d'incubation.

9. Procédé selon l'une des revendications 7 à 9 caractérisé en ce la fluorescence émise est convertie en LCH₄.Kg⁻¹ de matière brute, notamment selon la norme BMP (potentiel méthane) d'après un calcul comprenant les étapes suivantes :
- conversion des profils d'intensité d'absorbance-fluorescence ou des profils de vitesses instantanées en gC-Acétate.Kg⁻¹ à l'aide de l'équation mathématique issue de la régression linéaire de la courbe de calibration, ou
- référence à une base de données constituée d'échantillons avec valeur connue en gC-Acétate.Kg⁻¹ et BMP en LCH₄.Kg⁻¹ de matière brute pour prédire le potentiel méthane en LCH₄.Kg⁻¹ de matière brute.

10. Procédé selon la revendication 9 **caractérisé en ce que** le potentiel méthane d'échantillons inconnus est directement estimé en LCH₄.Kg⁻¹ de matière brute à partir de la courbe de calibration.

11. Procédé selon l'une quelconque des revendications précédentes **caractérisé en ce qu'il** met en oeuvre un kit comprenant au moins une microplaque, au moins un bioréactif fluorescent et/ou colorimétrique et des solutions-étalons et/ou de contrôle.

12. Procédé selon l'une quelconque des revendications précédentes **caractérisé en ce que** l'étape d'analyse de l'absorbance-fluorescence est mise en oeuvre par un système comprenant un lecteur de fluorescence adapté au format microplaque et des moyens de calculs agencés pour mettre en oeuvre ledit procédé.
